# EUROPEAN PATENT APPLICATION

(11) **EP 3 770 172 A1**
(43) Date of publication of application: **27.01.2021**
(21) Application number: 19771175.7
(22) Date of filing: 19.03.2019
(51) Int. Cl.: C07K 16/28, A61K 31/573, A61K 39/395, A61K 45/06, A61P 35/00, A61K 39/00

(54) **ANTI-VEGFR-2 ANTIBODY**

(30) Priority: 19.03.2018 KR 20180031677
(71) Applicant: Pharmabcine Inc., Yuseong-gu Daejeon 34047 (KR)
(72) Inventor: LEE, Weon-Sup, Daejeon 34120 (KR); LEE, Seon-Young, Seoul 05070 (KR); YOO, Jin-San, Daejeon 34124 (KR)
(74) Representative: Rückerl, Florian
(86) International application number: PCT/KR2019/003198
(87) International publication number: WO 2019/182333

(57) **Abstract**

The present invention relates to a pharmaceutical composition comprising an anti-VEGFR-2 antibody. The pharmaceutical composition of the present invention can treat, prevent, or alleviate cerebral edema. For patients who are administered a steroid agent to treat, prevent, or alleviate cerebral edema, the pharmaceutical composition of the present invention also allows a reduction in the administration dosage and/or duration of administration the steroid agent. In addition, the pharmaceutical composition of the present invention can significantly prolong a survival time of a patient suffering from cerebral edema or glioblastoma.

## Description

### [Technical Field]

The present invention relates to a pharmaceutical composition comprising an anti-VEGFR-2 antibody. The pharmaceutical composition of the present disclosure can treat, prevent, or alleviate cerebral edema. For patients administering a steroid agent in order to treat, prevent, or alleviate cerebral edema, the pharmaceutical composition of the present disclosure also allows a reduction in the dosage and/or duration of administration of the steroid agent. In addition, the pharmaceutical composition of the present disclosure can significantly prolong a survival time of a patient suffering from cerebral edema or glioblastoma.

### [Background Art]

Cerebral edema is one of the symptoms associated with brain tumor, glioblastoma, etc., and is a vasogenic edema that appears as the blood brain barrier collapses and body fluid accumulates in the extracellular space. In addition, cerebral edema has been reported as a result of traumatic brain injury, intracerebral hemorrhage, stroke, ischemic stroke, encephalitis, cerebral meningitis, or brain infection caused by viruses or bacteria.

Cerebral edema causes various neurological symptoms such as dizziness and headache, and further increases brain pressure, leading to death, so its severity is very high. In addition, edema often occurs when lymph node removal surgery is performed to prevent cancer metastasis in cancer patients, which leads to a significant reduction in the quality of life of cancer patients.

Drugs often used to alleviate or reduce such cerebral edema include osmotic agents, diuretics, glycerols, steroid agents, etc.

The representative example of osmotic agents is mannitol. However, when administering the mannitol for a long time, it may result in electrolyte dysfunction, serum osmotic concentration's increase, cardiopulmonary failure, renal failure and various nervous system problems.

It was reported that in an animal experimental model, the diuretics increase blood osmotic concentration to alleviate cerebral edema. However, there has not been reported about clinical research.

It was reported that the glycerols improve cerebral blood flow, and edema is reduced in cerebral infarction animal model. However, when rapidly administering glycerols to a subject, there are problems such as hemocytolysis, hemoglobinuria, renal failure.

Regarding the steroid agents, it was recently reported that corticosteroids inhibit a pathway of vascular endothelial growth factor (VEGF) to decrease tumor vascular permeability and suppress edema. Accordingly, corticosteroids have been often used as steroid agents for inhibiting vasogenic edema.

The corticosteroids include dexamethasone, cortisol, prednisone, prednisolone, methylprednisolone, triamcinolone, corticosterone, desonide, etc. Among them, dexamethasone is often used since it has better effect and longer half life compared to other corticosteroids. However, when administering dexamethasone in a high amount or for a long time, there are various side effects such as loss of muscle mass, increased risk of fractures, occurrence of diabetes and high blood pressure, immunity weakening, etc.

As described above, there is not yet an adequate therapy to treat or improve cerebral edema. Steroid agents such as dexamethasone, which are mainly used to alleviate cerebral edema, also exhibit serious side effects in a dose-dependent manner, and thus its application is limited.

### [Technical Problem]

As a result of the inventors' efforts to solve the problems in the prior art, the present invention was completed based on that the antibody according to the present disclosure can significantly treat or improve cerebral edema, and the dosage and/or the duration of a steroid agent administered to treat, prevent or improve cerebral edema can be significantly reduced.

### [Technical Solution]

One aspect of the present disclosure provides a pharmaceutical composition comprising an anti-VEGFR-2 antibody as an active ingredient.

The term "antibody" as used herein refers to an immunoglobulin molecule having immunological reactivity with a specific antigen or a protein molecule that serves as a receptor to specifically recognize an antigen. Therefore, in the present disclosure, "antibody" is used in a broad sense, and includes all of a polyclonal antibody, a monoclonal antibody, a whole antibody (an antibody consisting of at least two heavy chains and two light chains interconnected by a disulfide bond) and an antibody fragment. The whole antibodies include IgA, IgD, IgE, IgM and IgG. In addition, the IgG may include IgG1, IgG2, IgG3, and IgG4 as subtypes. The term "antibody fragment" refers to an antigen-binding fragment or analog of an antibody that has at least a portion of the binding specificity of the parent antibody and includes a portion (e.g., one or more CDRs) of the antigen binding region or variable regions of the parent antibody. The antibody fragment may be, for example, Fab, Fab', F(ab')2, Fv fragment, scFv, unibody, diabody, linear antibody, nanobody, domain antibody, or a multispecific antibody formed from antibody fragment.

The term "VEGFR" as used herein refers to a receptor of vascular endothelial growth factor, and includes VEGFR-1, VEGFR-2 and/or VEGFR-3. The biological function of VEGF, which enhances the growth activity of vascular endothelial cells, is mediated through the VEGF receptor having high affinity for VEGF, which is selectively expressed in endothelial cells during tumor formation.

The term "anti-VEGFR-2 antibody" as used herein refers to an antibody that neutralizes or inhibits the activity of VEGFR-2 by specifically binding to VEGFR-2 among subtypes of VEGF receptor. Specifically, the anti-VEGFR-2 antibody may be a human antibody targeting human VEGFR-2 (KDR) and also having reactivity to flk-1 derived from mouse or rat.

The term "human antibody" as used herein refers to an antibody having variable regions in which the framework and CDR regions are derived from human immunoglobulin sequences, and is also referred to as "fully human antibody". In the present disclosure, human antibodies may have amino acid residues that are not encoded by human-derived immunoglobulin sequences, e.g. mutations introduced by random or site-specific mutagenesis in vitro or by somatic mutation in vivo.

In one embodiment according to the present disclosure, the anti-VEGFR-2 antibody may be an antibody comprising a light chain variable region comprising light chain CDR1 of SEQ ID NO: 1, light chain CDR2 of SEQ ID NO: 2 and light chain CDR3 of SEQ ID NO: 3, and a heavy chain variable region comprising heavy chain CDR1 of SEQ ID NO: 4, heavy chain CDR2 of SEQ ID NO: 5 and heavy chain CDR3 of SEQ ID NO: 6. In addition, the anti-VEGFR-2 antibody may be preferably an antibody comprising a light chain variable region of SEQ ID NO: 7 and a heavy chain variable region of SEQ ID NO: 8. In addition, the anti-VEGFR antibody may be more preferably an TTAC-0001 antibody, or an antibody that specifically binds to the same epitope as the epitope to which the TTAC-0001 antibody binds. The TTAC-0001 antibody, also referred to as TANIBIRUMAB, is described in International Application WO 2008/153237 of the applicant (the entire contents of which are incorporated herein by reference) and the like, the disclosure of which is incorporated herein in its entirety by reference.

The pharmaceutical composition of the present disclosure is used to treat, prevent or alleviate cerebral edema. The cerebral edema may be caused by one or more factors such as brain tumor, glioblastoma, traumatic brain injury, intracerebral hemorrhage, stroke, ischemic stroke, encephalitis, cerebral meningitis, brain infection caused by viruses or bacteria, but is not limited thereto. The cerebral edema may be a peritumoral edema.

The term "treatment" or "treating" as used herein refers to any action in which symptoms of cerebral edema are improved or cured or the size of cerebral edema is reduced by administration of the composition according to the present disclosure.

The term "prevention" or "preventing" as used herein refers to any action in which cerebral edema is inhibited or delayed by administration of the composition according to the present disclosure.

The term "alleviation" or "alleviating" as used herein refers to any action in which the size or degree of cerebral edema is reduced, improved, or progression is delayed by the administration of the composition according to the present disclosure.

The pharmaceutical composition according to the present disclosure may comprise the anti-VEGFR antibody alone, or may additionally comprise one or more pharmaceutically acceptable carriers, excipients or diluents.

The Pharmaceutically acceptable carriers may include, for example, carriers for oral administration or non-oral administration. The carriers for oral administration may include, for example, lactose, starch, cellulose derivatives, magnesium stearate, stearic acid, etc. The carriers for non-oral administration may include, for example, water, suitable oils, saline, aqueous glucose, glycols, etc. In addition, the composition may comprise stabilizing agents or preserving agents. The suitable stabilizing agents may include, for example, antioxidants such as sodium bisulfate, sodium sulfite or ascorbic acid. The suitable preserving agents may include, for example, benzalkonium chloride, methyl- or propyl-paraben, chlorobutanol, etc. Other pharmaceutically acceptable carriers may be those disclosed in the literature "Remington's Pharmaceutical Sciences, 19th ed., Mack Publishing Company, Easton, PA, 1995*."*

The pharmaceutical composition of the present disclosure may be administered to mammals including human using any methods. For example, it may be administered orally or parenterally. The parenteral administration may include, but are not limited to, intravenous, intramuscular, intraarterial, intramarrow, intradural, intracardial, percutaneous, subcutaneous, intraperitoneal, intranasal, intraintestinal, topical, sublingual or rectal administration. For example, the pharmaceutical composition of the present disclosure may be prepared in a formulation for injection, and administered by a method of lightly pricking the skin with a 30 gauge thin injection needle, or by applying it directly to the skin.

The pharmaceutical composition of the present disclosure may be prepared into formulations for oral or parenteral administration, depending on the administration route as described in the above.

The formulation for oral administration may be prepared in the form of powders, granules, tablets, pills, sugar-coated pills, capsules, liquids, gels, syrups, slurries, suspensions, etc., using methods known in the art. For example, the active ingredient mixed with a solid excipient may be pulverized to add a suitable adjuvant, and then processed into a granule mixture to obtain a tablet or a sugar-coated tablet for oral administration. Examples of suitable excipients may include, but are not limited to, sugars including lactose, dextrose, sucrose, sorbitol, mannitol, xylitol, erythritol, maltitol, etc., starches including corn starch, wheat starch, rice starch, potato starch, etc., celluloses including cellulose, methyl cellulose, sodium carboxymethylcellulose, hydroxypropylmethylcellulose, etc., and fillers such as gelatin, polyvinyl pyrrolidone, etc. Optionally, disintegrating agents such as crosslinked polyvinyl pyrrolidone, agar, alginic acid or sodium alginate may be added. In addition, the pharmaceutical composition of the present disclosure may further comprise anticoagulants, lubricants, wetting agents, aromatic agents, emulsifiers and preservatives, etc.

The formulation for parenteral administration may be prepared in the form of injections, creams, lotions, ointments, oils, moisturizers, gels, aerosols, nasal inhalers using methods known in the art. These administration forms may refer to those disclosed in the literature "Remington's Pharmaceutical Science, 15th Edition, 1975. Mack Publishing Company, Easton, Pennsylvania 18042, Chapter 87: Blaug, Seymour*."*

The total effective dose of the pharmaceutical composition according to the present disclosure may be administered to a subject in a single dose, or in multiple doses through a fractionated treatment protocol. For example, the total dose of the pharmaceutical composition according to the present disclosure may be about 0.01µg to 1,000mg per 1kg body weight of a patient per day, preferably 0.1 µg to 100mg. However, the appropriate dose of the pharmaceutical composition according to the present disclosure may be determined considering various factors such as administration route, times administered, patient's age, body weight, health, gender, severity of disease, diet, excretion rate, etc. by a person having ordinary skill in the art. There is no particular limit to the dosage form, administration route and administration method, so long as the pharmaceutical composition shows the effect of the invention. The contents of active ingredient anti-VEGFR-2 antibody in the pharmaceutical composition may vary depending on symptom of cerebral edema. Preferably, the anti-VEGFR-2 antibody according to the present disclosure may be contained in the composition in an amount of about 8mg to 24mg per 1kg body weight of a patient every week. The examples described hereafter showed that the anti-VEGFR-2 antibody in an amount of 8mg to 24mg per 1kg body weight of a patient every week can significantly reduce the size of edema in the subject administered, and it can be administered in an amount of 24mg per 1kg body weight of a patient every week in order to increase the effect of reducing the size of edema.

In addition, the pharmaceutical composition of the present disclosure may be administered as an individual therapeutic agent or in combination with other therapeutic agents. It may be administered simultaneously, individually or sequentially or with other therapeutic agents. The other therapeutic agents may be those which have already known in the art as having an effect of treating or alleviating cerebral edema, and may be, but are not limited to, osmotic agents, diuretics, glycerols, steroid agents, etc. The steroid agent may be corticosteroid which may include dexamethasone, cortisol, prednisone, prednisolone, methylprednisolone, triamcinolone, corticosterone, desonide, etc. Furthermore, the other therapeutic agents include all anticancer therapies such as radiation therapy besides drug therapy.

When administering the pharmaceutical composition of the present disclosure in combination with other therapeutic agents, an anti-VEGFR-2 antibody contained in the composition and other therapeutic agents such as a steroid agent may be formulated in the form separated in each container, or together in a single container.

Another aspect of the present invention provides a method for treating or preventing cerebral edema, comprising administering an anti-VEGFR-2 antibody to a subject.

In the method for treating or preventing cerebral edema according to the present disclosure, each term has the same meaning as described in the pharmaceutical composition for treating or preventing cerebral edema, unless otherwise specified.

The term "subject" includes any human or non-human animal. The term "non-human animal" may be a vertebrate such as non-human primates, sheep, dogs, and rodents such as mice, rats and guinea pigs. The subject may preferably be a human. The term "subject" herein is used interchangeably with "individual" and "patient".

The treatment of cerebral edema may be, for example, to inhibit the growth or expansion of the cerebral edema, to maintain the size of the cerebral edema, to reduce the size of the cerebral edema, or to eliminate the cerebral edema, compared to the untreated subject. For example, it means that the size of cerebral edema before treatment may be reduced by about 10% or more, about 20% or more, about 30% or more, about 40% or more, about 50% or more, about 60% or more, about 70% or more, about 80% or more, or about 90% or more after treatment.

In the method for treating or preventing cerebral edema according to the present disclosure, the anti-VEGFR-2 antibody may be administered to a subject simultaneously, sequentially, or individually with other therapeutic agents such as a steroid agent. The "simultaneous" administration means that the anti-VEGFR-2 antibody and other therapeutic agents are administered at one time through the same injection method. The "sequential" administration means that the anti-VEGFR-2 antibody and other therapeutic agents are administered separately, but relatively continuously, allowing the minimum possible time as the time consumed in the administration interval.. The "individual" administration means that the anti-VEGFR-2 antibody and other therapeutic agents are administered at regular intervals. The administration method of anti-VEGFR-2 antibody and other therapeutic agents may be appropriately selected by those skilled in the art considering therapeutic effects and side effects of the patient.

Another aspect of the present invention provides a pharmaceutical composition for reducing dosage, duration, or dosage and duration of administration of a steroid agent in a patient receiving the steroid agent to treat, prevent or alleviate cerebral edema. The pharmaceutical composition contains an anti-VEGFR-2 antibody as an active ingredient.

In the pharmaceutical composition for reducing one or more of the dosage and duration of administration of the steroid agent according to the present disclosure, each term has the same meaning as described in the pharmaceutical composition for the treatment or prevention of cerebral edema unless otherwise stated.

The dosage of the steroid agent is an amount that a patient with cerebral edema is administered to treat or ameliorate cerebral edema, or an amount that a subject at risk of having cerebral edema or suspected of having cerebral edema is administered to prevent the occurrence of cerebral edema. The dosage of the steroid agent may be a single dose or multiple dose, and may vary depending on symptoms of cerebral edema. The pharmaceutical composition according to the present disclosure can significantly reduce or minimize the dosage of the steroid agent.

The duration of administration of the steroid agent means the entire period during which a patient with cerebral edema is administered to treat or ameliorate cerebral edema, or which a subject at risk of having cerebral edema or suspected of having cerebral edema is administered to prevent the occurrence of cerebral edema. The duration of administration of the steroid agent may vary depending on the symptoms of cerebral edema. The pharmaceutical composition according to the present disclosure can significantly reduce or minimize the duration of administration of the steroid agent.

In addition, the composition makes it possible to obtain an desired effect of treating, preventing or improving cerebral edema even if the route of administration of steroid agent is changed from intravenous to oral administration. This is very advantageous because the patient can increase a convenience and compliance with the steroid agent.

Another aspect of the present invention provides a method of reducing one or more of the dosage and duration of administration of a steroid agent, comprising administering an anti-VEGFR-2 antibody to a subject receiving a steroid agent to treat, prevent or improve cerebral edema.

This method can change the route of administration of the steroid agent from intravenous administration to oral administration.

In the method, each term has the same meaning as described in the pharmaceutical composition for treating or preventing cerebral edema, the method for treating or preventing cerebral edema, and the pharmaceutical composition for reducing dosage, duration, or dosage and duration of administration of a steroid agent, unless otherwise specified.

The anti-VEGFR-2 antibody according to the present disclosure can treat, prevent or alleviate cerebral edema. More specifically, it may inhibit expansion, swelling or growth of the cerebral edema, maintain a size of the cerebral edema, significantly reduce a size of the cerebral edema, or eliminate the cerebral edema. In addition, the antibody according to the present disclosure exhibits an effect of improving the midline shift of the brain caused by cerebral edema.

Even if a patient who has received a drug such as a steroid agent in order to treat or alleviate cerebral edema is no longer administered the steroid agent or is administered the reduced dosage of steroid agent, the anti-VEGFR-2 antibody according to the present disclosure can significantly reduce cerebral edema. As a result, while achieving the desired cerebral edema treatment or improvement effect, it is possible to minimize or avoid various side effects such as loss of muscle mass, increased risk of fractures, diabetes and hypertension, or weakened body immune function that occur with high doses or long-term administration of drugs such as a steroid agent.

In addition, even if the dosage and/or duration of administration of the steroid agent is reduced and the route of administration thereof is changed from intravenous to oral administration, the treatment, prevention or improvement effect of cerebral edema can be obtained. It is very advantageous in that the patient can increase the convenience and compliance with the steroid agent.

In addition, when the antibody according to the present disclosure is administered to a patient with cerebral edema, particularly a patient with recurrent glioblastoma, the overall survival time can be significantly extended.

The articles "a" and "an" are used herein to refer to one or to more than one (i.e. to at least one) of the grammatical object of the article. By way of example, "an ingredient" means one ingredient or more than one ingredient. The term "A, B and/or C" is used herein to refer to A, or B, or C, or A and B, or A and C, or B and C, or A, B and C.

### [Brief Description of Figures]

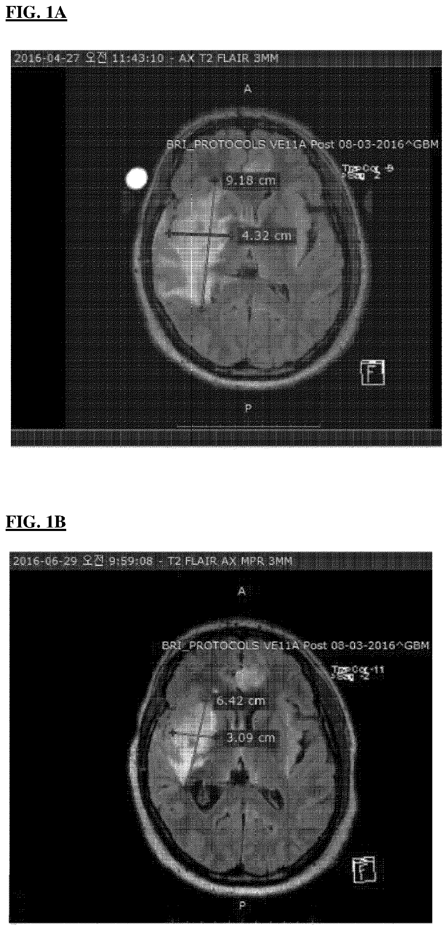

FIGS. 1A and 1B are MRI photographs of the brain of patient in Example 3. Specifically, FIG. 1A is a picture taken when only a steroid agent is administered, and FIG. 1B is a picture taken after reducing the dosage of the steroid agent to 50% or less while administering the antibody according to the present disclosure.

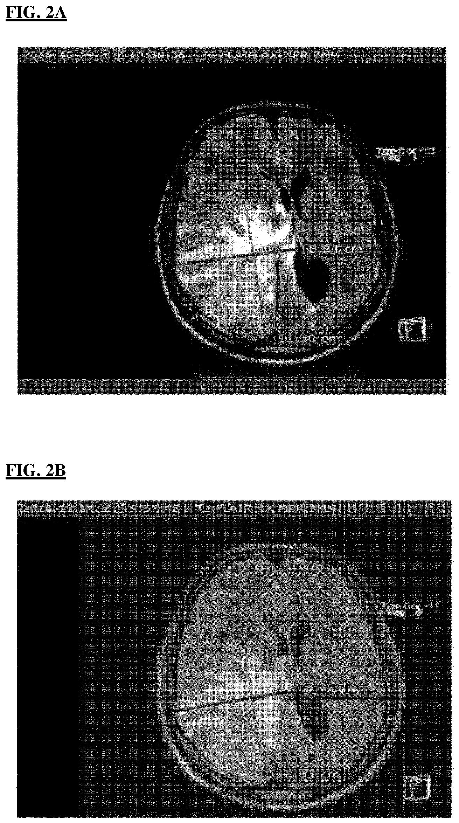

FIGS. 2A and 2B are MRI photographs of the brain of patient in Example 7. Specifically, FIG. 2A is a picture taken before administration of the antibody according to the present disclosure, and FIG. 2B is a picture taken after administration of the antibody according to the present disclosure.

### [EXAMPLES]

In the following, exemplary embodiments of the inventive concept will be explained in further detail with reference to examples. However, the following examples are meant to exemplify the present invention, and the scope of the invention is not restricted by these examples.

For this experiment, patients with severe cerebral edema having recurrent glioblastoma and life expectancy of 12 weeks or more were recruited. In order to clearly confirm the effect of the antibody according to the present disclosure, patients who have been treated with an agent targeting VEGF were excluded. The information of the patients selected accordingly is shown in Table 1 below.

**Table 1**

| | **Patient's gender** | **Patient's age** | **Number of recurrence of glioblastoma** |
|---|---|---|---|
| **Example 1** | male | 47 | twice |
| **Example 2** | male | 49 | once |
| **Example 3** | male | 41 | once |
| **Example 4** | male | 53 | twice |
| **Example 5** | female | 50 | twice |
| **Example 6** | female | 60 | once |
| **Example 7** | male | 48 | once |
| **Example 8** | male | 51 | once |
| **Example 9** | female | 50 | twice |
| **Example 10** | female | 64 | twice |
| **Example 11** | male | 68 | once |
| **Example 12** | male | 48 | once |

In this experiment, as the anti-VEGFR-2 antibody, a TTAC-0001 antibody including the light chain variable region of SEQ ID NO: 7 and the heavy chain variable region of SEQ ID NO: 8 was used. The dosage and administration interval of the TTAC-0001 antibody are shown in Table 2 below, respectively.

**Table 2**

| | **Dosage of TTAC-0001 antibody** | **Administration interval of TTAC-0001 antibody** |
|---|---|---|
| **Example 1** | 8mg/kg | Once a week injection |
| | | (3 injections + 1 week observation = 1 cvcle) |
| **Example 2** | 8mg/kg | Once a week injection |
| | | (3 injections + 1 week observation = 1 cvcle) |
| **Example 3** | 12mg/kg | Once a week injection |
| | | (3 injections + 1 week observation = 1 cvcle) |
| **Example 4** | 12mg/kg | Once a week injection |
| | | (3 injections + 1 week observation = 1 cycle) |
| **Example 5** | 12mg/kg | Once a week injection |
| | | (4 injections = 1 cycle) |
| **Example 6** | 12mg/kg | Once a week injection |
| | | (4 injections = 1 cycle) |
| **Example 7** | 12mg/kg | Once a week injection |
| | | (4 injections = 1 cycle) |
| **Example 8** | 8mg/kg | Once a week injection |
| | | (3 injections + 1 week observation = 1 cycle) |
| **Example 9** | 12mg/kg | Once a week injection |
| | | (3 injections + 1 week observation = 1 cycle) |
| **Example 10** | 12mg/kg | Once a week injection |
| | | (4 injections = 1 cycle) |
| **Example 11** | 12mg/kg | Once a week injection |
| | | (4 injections = 1 cycle) |
| **Example 12** | 12mg/kg | Once a week injection |
| | | (4 injections = 1 cycle) |

During the period of administration of TTAC-0001 antibody to the patients of Examples 1 to 12, symptoms such as fatigue and mild headache were observed in some patients, but these were not considered as serious side effects according to the opinion of the clinician. In addition, side effects caused by administration of TTAC-0001 antibody, such as a rapid decrease in body weight, were not observed. This shows that the antibody according to the present disclosure is not toxic to the human body and can be used safely.

MRI scans were performed every 8 weeks to obtain MRI images. In MRI images, the longest and shortest diameters were measured for cerebral edema and tumor, respectively, and the volume thereof was calculated. The quantitative data obtained as a result are summarized and shown in Table 3 below together with the contents of qualitative analysis.

**Table 3**

| | **MRI date** | **Longest diameter of peritumoral edema (cm)** | **Volume of peritumoral edema (cm³)** | **Analysis** |
|---|---|---|---|---|
| **Example 1** | Feb 17, 2016 | 7.90 | 1529.8 | Edema increases in size. |
| | Mar 31, 2016 | 10.67 | 1797.4 | |
| **Example 2** | Feb 24, 2016 | 10.03 | 1355.6 | From Feb 24, 2016 to Dec 07, 2016, the size of edema continuously decreases. |
| | Apr 27, 2016 | 8.22 | 859.0 | |
| | Jun 22, 2016 | 8.04 | 809.3 | |
| | Aug 18, 2016 | 8.71 | 901.9 | |
| | Oct 12, 2016 | 7.39 | 772.4 | |
| | Dec 07, 2016 | 6.71 | 666.0 | |
| | Feb 22, 2017 | 7.26 | 728.2 | |
| **Example 3** | Apr 27, 2016 | 9.18 | 706.5 | Edema decreases in size. |
| | Jun 29, 2016 | 6.42 | 415.6 | |
| **Example 4** | Jul 13, 2016 | 11.20 | 1098.4 | Edema decreases in size. |
| | Sep 08, 2016 | 9.85 | 1050.6 | |
| **Example 5** | Aug 18, 2016 | 9.13 | 1061.7 | No significant change in edema size. |
| | Oct 19, 2016 | 8.85 | 917.3 | |
| | Dec 15, 2016 | 9.20 | 1011.9 | |
| | Feb 08, 2017 | 9.15 | 1065.3 | |
| | Apr 05, 2017 | 9.07 | 1000.9 | |
| **Example 6** | Sep 15, 2016 | 7.73 | 840.1 | No significant change in edema size |
| | Nov 16, 2016 | 7.11 | 846.2 | |
| **Example 7** | Oct 19, 2016 | 11.30 | 1291.8 | Edema decreases in size, and the midline shift is improved. |
| | Dec 14, 2016 | 10.33 | 1221.0 | |
| **Example 8** | Feb 09, 2016 | 5.93 | 424.5 | Edema increases in size. |
| | Mar 31, 2016 | 8.31 | 650.7 | |
| **Example 9** | Jul 07, 2016 | 7.95 | 734.0 | Edema increases in size. |
| | Sep 01, 2016 | 8.10 | 812.5 | |
| | Oct 31, 2016 | 8.77 | 852.0 | |
| **Example 10** | Aug 12, 2016 | 7.52 | 1102.2 | Edema decreases in size. |
| | Oct 06, 2016 | 6.21 | 930.7 | |
| **Example 11** | Sep 02, 2016 | 11.72 | 850.3 | No significant change in edema size. |
| | Oct 27, 2016 | 11.89 | 855.4 | |
| **Example 12** | Sep 08, 2016 | 8.46 | 948.9 | No significant change in edema size. |
| | Nov 03, 2016 | 8.93 | 906.3 | |

As shown in Table 3, among 12 patients with cerebral edema treated with the antibody according to the present disclosure, 5 patients had reduced cerebral edema size, and 4 patients maintained the size of cerebral edema without significant change. In the remaining 3 patients, the tumor progressed or its size increased, and accordinly the size of the edema also increased. That is, for nearly half of the patients, the size of cerebral edema was significantly reduced, and side effects caused by cerebral edema, such as midline shift, were improved.

Hereinafter, exemplary embodiments are described in more detail.

Patients in Examples 1 and 8 showed an increase in the size of the cerebral edema. In this regard, the tumor size of the patient of Example 1 increased by about 44.2% from 267 cm³ to 385 cm³ in the second MRI scan compared to the first MRI scan. In addition, the tumor size of the patient of Example 8 increased by about 137.1% from 735 cm³ to 1743 cm³ in the second MRI scan compared to the first MRI scan. It is confirmed from the opinion of the clinician that the increase in the size of the cerebral edema in the patients in Examples 1 and 8 is accompanied by the result of increasing the size of the peritumoral edema according to the tumor progression in brain.

In the case of Example 7, it was especially observed that the midline shift of the brain was improved as the size of edema was reduced by administration of the antibody according to the present disclosure. This can be confirmed through comparison of FIGS. 2A and 2B. Midline shift is also called median deviation, centerline shift, midline transposition, etc., and it indicates that the line crossing the center line of the brain leans toward the area where there is no abnormality, due to high intracranial pressure (ICP), traumatic brain injury, stroke, cerebral hemorrhage, brain tumor, hematoma, etc. Midline shift is considered as a poor prognosis, and is generally associated with distortion of the brain stem, which can cause serious dysfunction such as abnormal posturing or failure to constrict the pupil in response to light. Usually, if the midline shift exceeds 5 mm, immediate surgery is recommended. Such midline shift of brain can obtain a significant improvement effect non-invasively by the antibody according to the present disclosure.

In the case of Example 3, in order to alleviate the cerebral edema of a patient with recurrent glioblastoma, 8 mg of dexamethasone as a steroid agent was administered intravenously on March 29, 2016. A photograph of the brain taken by MRI on April 27, 2016 is shown in FIG. 1A, and a white portion shown on the left side of FIG. 1A indicates cerebral edema. From May 4, 2016, TTAC-0001 antibody was administered at a dose of 12 mg/kg over 2 cycles. In addition, from May 12, 2016 to June 15, 2016, the dosage of dexamethasone was reduced to 4 mg, and the route of administration was changed from intravenous to oral. Subsequently, from June 16, 2016 to June 29, 2016, the dosage of dexamethasone was further reduced to 3 mg, and the route of administration was still oral. A photograph of the brain taken by MRI on June 29, 2016 is shown in FIG. 1B, and the white portion shown on the left of FIG. 1B indicates the cerebral edema.

As a result, it was confirmed that the volume of the cerebral edema of patient in Example 3 was remarkably reduced from 706.5 cm³ to 415.6 cm³, unpredictable by those of ordinary skill in the art. This shows that the size of the cerebral edema, which was not reduced even though dexamethasone mainly used so far was administered at a high dose, can be reduced after administration of the anti-VEGFR-2 antibody of the present disclosure.

Moreover, when the administration of the anti-VEGFR-2 antibody of the present disclosure was started, even if the dosage of dexamethasone was reduced by 50% or more while oral administration for improved patient convenience was performed instead of intravenous administration for rapid effect achievement, the desired cerebral edema size reduction effect could be obtained. It means that when using the antibody according to the present disclosure with a steroid agent, it is possible to reduce or avoid side effects associated with high doses and/or long-term use of the steroid agent, such as loss of muscle mass, increased risk of fractures, diabetes and high blood pressure, and weakening of the body's immune function, and also that patient convenience can be improved according to changes in route of administration.

As in Example 3, in Example 10, the dosage of dexamethasone was increased in the order of dexamethasone 6mg orally on August 5, 2016, and dexamethasone 8mg oral administration on August 17, 2016. And then, on August 18, 2016 when TTAC-0001 antibody was administered, the oral dose of dexamethasone was reduced to 4 mg. Subsequently, dexamethasone 4mg was administered orally on August 25, 2016, August 31, 2016, September 7, 2016, September 14, 2016, September 21, 2016, and October 5, 2016. Although the amount of dexamethasone administered to alleviate cerebral edema was reduced while administering the antibody according to the present disclosure, the volume of the cerebral edema of patient in Example 10 was confirmed to decrease from 1102.2 cm³ to 930.7 cm³.

Meanwhile, the median overall survival time (mOS) was predicted for the Example's patients based on a regressive segmentation analysis method for recurrent glioblastoma patients *(*Prognostic factors for survival in adult patients with recurrent glioma enrolled onto the new approaches to brain tumor therapy CNS consortium phase I and II clinical trials, Carson et al., 2007), and the actual overall survival time (OS) of the Example's patients was recorded.

As a result, the patient of Example 5 is expected to have a median overall survival time (mOS) of 4.9 months, the patient of Example 9 is expected to have a median overall survival time (mOS) of 4.9 months, and the patient of Example 2 is expected to have a median overall survival time of 10.4 months.

Corresponding to this, the actual overall survival time for Example 5's patient was 17.3 months, the actual overall survival time for Example 9's patient was 12.9 months, and the actual overall survival time for Example 2's patient was 15.9 months.

Thus, when the antibody according to the present disclosure is administered to a recurrent glioblastoma patient with cerebral edema, the prolonged overall survival time is concluded to be very significant compared to the overall survival time predicted based on the regression segmentation analysis method.

## Claims

1. A pharmaceutical composition comprising an anti-VEGFR-2 antibody that specifically binds to VEGFR-2 to neutralize an activity of VEGFR-2, for treating, preventing, or alleviating cerebral edema.

2. The pharmaceutical composition according to claim 1, wherein the anti-VEGFR-2 antibody comprises:
a light chain variable region comprising light chain CDR1 of SEQ ID NO: 1, light chain CDR2 of SEQ ID NO: 2, and light chain CDR3 of SEQ ID NO: 3; and
a heavy chain variable region comprising heavy chain CDR1 of SEQ ID NO: 4, heavy chain CDR2 of SEQ ID NO: 5, and heavy chain CDR3 of SEQ ID NO: 6.

3. The pharmaceutical composition according to claim 1, wherein the anti-VEGFR-2 antibody comprises:
a light chain variable region of SEQ ID NO: 7; and
a heavy chain variable region of SEQ ID NO: 8.

4. The pharmaceutical composition according to claim 1, wherein the anti-VEGFR-2 antibody is a TTAC-0001 antibody or an antibody specifically binding to the same epitope as the epitope bound by the TTAC-0001 antibody.

5. The pharmaceutical composition according to claim 1, wherein the cerebral edema is a glioblastoma surrounding edema.

6. The pharmaceutical composition according to claim 1, wherein the composition further comprises one or more selected from the group consisting of osmotic agents, diuretics, glycerols and steroid agents.

7. The pharmaceutical composition according to claim 6, wherein the steroid agent is one or more selected among dexamethasone, cortisol, prednisone, prednisolone, methylprednisolone, triamcinolone, corticosterone and desonide.

8. The pharmaceutical composition according to claim 1, wherein the composition prolongs a survival time of a subject suffering from cerebral edema.

9. A pharmaceutical composition for reducing one or more of dosage and duration of administration of a steroid agent in a subject receiving the steroid agent to treat, prevent or alleviate cerebral edema, comprising an anti-VEGFR-2 antibody that specifically binds to VEGFR-2 to neutralize an activity of VEGFR-2

10. The pharmaceutical composition according to claim 9, wherein the anti-VEGFR-2 antibody comprises:
a light chain variable region comprising light chain CDR1 of SEQ ID NO: 1, light chain CDR2 of SEQ ID NO: 2, and light chain CDR3 of SEQ ID NO: 3; and
a heavy chain variable region comprising heavy chain CDR1 of SEQ ID NO: 4, heavy chain CDR2 of SEQ ID NO: 5, and heavy chain CDR3 of SEQ ID NO: 6.

11. The pharmaceutical composition according to claim 9, wherein the anti-VEGFR-2 antibody comprises:
a light chain variable region of SEQ ID NO: 7; and
a heavy chain variable region of SEQ ID NO: 8.

12. The pharmaceutical composition according to claim 9, wherein the anti-VEGFR-2 antibody is a TTAC-0001 antibody or an antibody specifically binding to the same epitope as the epitope bound by the TTAC-0001 antibody.

13. The pharmaceutical composition according to claim 9, wherein the subject is a subject suffering from glioblastoma.

14. The pharmaceutical composition according to claim 9, wherein the route of administration of steroid agent is oral administration.

15. The pharmaceutical composition according to claim 9, wherein the composition prolongs a survival time of the subject.
